# EUROPEAN PATENT SPECIFICATION

(11) **EP 4 062 951 B1**
(45) Date of publication and mention of the grant of the patent: **23.07.2025**
(21) Application number: 20889312.3
(22) Date of filing: 17.11.2020
(51) Int. Cl.: A61L 27/54, A61L 27/52, A61L 27/38, A61L 27/50, A61L 27/20

(54) **INJECTABLE HYDROGEL COMPOSITION CAPABLE OF CAPTURING ENDOGENOUS PROGENITOR CELLS OR STEM CELLS, AND INDUCING VASCULAR DIFFERENTIATION OF CAPTURED CELLS**
INJIZIERBARE HYDROGELZUSAMMENSETZUNG ZUM EINFANGEN ENDOGENER VORLÄUFERZELLEN ODER STAMMZELLEN UND INDUZIERUNG DER VASKULÄREN DIFFERENZIERUNG VON EINGEFANGENEN ZELLEN
COMPOSITION D'HYDROGEL INJECTABLE POUVANT CAPTURER DES CELLULES PROGÉNITRICES OU DES CELLULES SOUCHES ENDOGÈNES ET INDUIRE UNE DIFFÉRENCIATION VASCULAIRE DES CELLULES CAPTURÉES

(30) Priority: 22.11.2019 KR 20190151219
(43) Date of publication of application: 28.09.2022
(73) Proprietor: Ajou University Industry-Academic Cooperation Foundation, Gyeonggi-do 16499 (KR)
(72) Inventor: KIM, Moon Suk, Suwon-si, Gyeonggi-do 16499 (KR); PARK, Seung Hun, Seoul 05747 (KR)
(74) Representative: Hutter, Anton
(86) International application number: PCT/KR2020/016135
(87) International publication number: WO 2021/101198

(56) References cited:
- KR-A- 20130 114 968
- KR-A- 20150 006 223
- KR-A- 20150 006 223
- KR-A- 20170 012 099
- KR-A- 20190 040 757
- KR-A- 20190 040 757
- SONG MYEONGJIN ET AL: "Regeneration of chronic myocardial infarction by injectable hydrogels containing stem cell homing factor SDF-1 and angiogenic peptide Ac-SDKP", BIOMATERIALS, ELSEVIER, AMSTERDAM, NL, vol. 35, no. 8, 28 December 2013 (2013-12-28), pages 2436 - 2445, XP028814363, ISSN: 0142-9612, DOI: 10.1016/J.BIOMATERIALS.2013.12.011
- SONG MYEONGJIN; JANG HWANSEOK; LEE JAEYEON; KIM JI HYUN; KIM SOO HYUN; SUN KYUNG; PARK YONGDOO: "Regeneration of chronic myocardial infarction by injectable hydrogels containing stem cell homing factor SDF-1 and angiogenic peptide Ac-SDKP", BIOMATERIALS, ELSEVIER, AMSTERDAM, NL, vol. 35, no. 8, 28 December 2013 (2013-12-28), AMSTERDAM, NL, pages 2436 - 2445, XP028814363, ISSN: 0142-9612, DOI: 10.1016/j.biomaterials.2013.12.011

## Description

### CROSS-REFERENCE TO RELATED APPLICATION

This application claims priority to and the benefit of Korean Patent Application No. 10-2019-0151219, filed on November 22, 2019.

### [Technical Field]

The present invention relates to an injectable hydrogel composition having the recruitment of endogenous progenitors or stem cells and the induction of vascular differentiation of recruited cells, and more specifically to an injectable hydrogel composition having the recruitment of endogenous progenitors or stem cells and the induction of vascular differentiation of recruited cells, which consists of: a first solution including anionic hyaluronic acid into which a vascular differentiation inducing factor is introduced; and a second solution including a cationic material, wherein a stem cell recruitment factor is further included in the first solution and/or the second solution, and wherein a hydrogel is formed by electrostatic interaction.

### [Background Art]

With the exception of cornea and cartilage, all tissue engineering substitute materials require vascular neural networks to supply nutrients and oxygen necessary for survival *in vivo.* Since angiogenesis in artificial tissues can take several weeks to occur spontaneously, the tissue develops necrosis during this time due to a lack of supply of essential nutrients. Therefore, the formation of blood vessels in regenerative medicine is a very important problem in the regeneration of damaged tissues or the development of artificial organs.

Angiogenesis is a process in which endothelial cells of existing blood vessels decompose, migrate, divide and differentiate the extracellular matrix (ECM) to form new capillaries, and it is involved in various physiological and pathological phenomena such as wound repair, embryogenesis, tumor formation, chronic inflammation, obesity and the like. The angiogenic process includes the proliferation and migration of vascular endothelial cells from the vessel wall to surrounding tissues in the direction of stimulation. Subsequently, various proteolytic enzymes are activated such that vascular endothelial cells infiltrate the basement membrane and form loops, and the formed loops are differentiated to form tubes.

In order to induce or promote angiogenesis, many studies have been conducted to induce differentiation of stem cells into vascular endothelial cells, but in fact, it has been mainly reported that angiogenesis of the host is induced by growth factors secreted from stem cells rather than stem cells themselves induce angiogenesis. In addition, it has been reported that among the cells produced by decomposing adipose tissue, a structural vascular fraction (SVF) can be transplanted into animals without culturing to differentiate into vascular endothelial cells. However, the method does not induce proliferation by subculture of adipose stem cells, and thus, the amount of vascular endothelial cells differentiated from adipose stem cells is very small, and in particular, since the proliferation and differentiation rates of differentiated vascular endothelial cells are low, the application thereof is limited.

Among the methods using stem cells, the method of using "self-regeneration" activates the endogenous progenitor cells/stem cells originally present in the patient's own body, thereby regenerating damaged organs and/or tissues to recover of their functions. Endogenous progenitor cells/stem cells can differentiate into different types of cells depending on the different types of cells in contact, the content of extracellular matrix and the microenvironment in which the cells are present, such as cytokines and growth factors.

In order to recruit endogenous progenitor cells/stem cells into the wound site or the tissue damaged site, stem cell recruitment factors can be used, and among them, substance P (SP), which is known as a stem cell recruitment factor, has been reported to be helpful in wound treatment, but since it is used in a simple solution state, it has the disadvantage that it does not stay for a long period of time in the tissue damaged site, and thus, the development of a method that can continuously release substance P from the damaged area is required.

Song et al. ("Regeneration of chronic myocardial infarction by injectable hydrogels containing stem cell homing factor SDF-1 and angiogenic peptide Ac-SDKP", Biomaterials, 2014 Mar; 35(8): 2436-45) discloses that the combination of stem cell homing factor (SDF-1) and angiogenic peptides (Ac-SDKP) injected with biomimetic hydroge promotes regeneration of cardiac function.

Accordingly, in the present invention, as a result of diligent efforts to develop a substance that can effectively recruit endogenous progenitor cells/stem cells and promote angiogenesis by inducing vascular differentiation at the same time, it was confirmed that when the vascular endothelial growth factor mimic peptide (VP), which is capable of differentiating progenitor/stem cells into vascular cells, was mixed with HA-VP, which was chemically introduced into anionic hyaluronic acid, and a cationic material, a hydrogel was formed by electrostatic interaction, and the present invention was completed by confirming that when the progenitor cell/stem cell recruitment factor (substance P) was physically supported and injected *in vivo,* the stem cell recruitment factor was released from the injected hydrogel such that endogenous progenitor cells/stem cells were recruited in the hydrogel, and the induction of angiogenesis was promoted by differentiation into vascular cells by VP which was chemically introduced into hyaluronic acid.

The invention is as defined in the independent claims. Preferred embodiments of the invention are recited in the dependent claims. Various other embodiments are included in the description, not all of which are encompassed by the claims, but are included to aid the reader's understanding of the claimed invention.

### [Disclosure]

### [Technical Problem]

An object of the present disclosure is to provide an injectable hydrogel composition having the recruitment of progenitor cells/stem cells and the induction of vascular differentiation of recruited cells.

### [Technical Solution]

In a first aspect, the invention provides an injectable hydrogel composition according to claims 1 or 2 having the recruitment of endogenous progenitors or stem cells and the induction of vascular differentiation of recruited cells, consisting of: a first solution comprising anionic hyaluronic acid into which a vascular differentiation inducing factor is introduced, which is prepared by reacting the vascular differentiation inducing factor and anionic hyaluronic acid in which a carboxylic acid functional group is activated; and a second solution comprising a cationic material, at least one selected from the group consisting of chitosan, cationic dextran, polyethyleneimine, polylysine and polyhistidine, wherein a stem cell recruitment factor, substance P, is further comprised in any one or more of the first solution and the second solution, wherein when the first solution and the second solution are mixed in ratio of 2:1 to 1:2, a hydrogel is formed by electrostatic interaction, wherein the vascular differentiation inducing factor is a vascular endothelial cell growth factor mimic peptide, and wherein the vascular endothelial cell growth factor mimic peptide comprises the amino acid sequence represented by SEQ ID NO: 1.

In order to achieve the aforementioned object, the present disclosure provides an injectable hydrogel composition having the recruitment of endogenous progenitors or stem cells and the induction of vascular differentiation of recruited cells, which consists of a first solution including anionic hyaluronic acid into which a vascular differentiation inducing factor is introduced; and a second solution including a cationic material, wherein a stem cell recruitment factor is further included in any one or more of the first solution and the second solution, and wherein when the first solution and the second solution are mixed, a hydrogel is formed by electrostatic interaction.

The anionic hyaluronic acid into which the vascular differentiation inducing factor is introduced may be prepared by reacting the vascular differentiation inducing factor and anionic hyaluronic acid in which a carboxylic acid functional group is activated.

The cationic material may be at least one selected from the group consisting of chitosan, cationic dextran, polyethyleneimine, polylysine and polyhistidine.

The stem cell recruitment factor is substance P.

According to a preferred exemplary embodiment of the present disclosure, the storage modulus of the hydrogel formed by mixing the first solution and the second solution may be 10 to 100 Pa.

In addition, the present invention provides the injectable hydrogel according to either claim 1 or 2, for use in tissue regeneration.

In one aspect, the present disclosure relates to an injectable hydrogel composition having the recruitment of endogenous progenitors or stem cells and the induction of vascular differentiation of recruited cells, which consists of a first solution including anionic hyaluronic acid into which a vascular differentiation inducing factor is introduced; and a second solution including a cationic material, wherein a stem cell recruitment factor is further included in any one or more of the first solution and the second solution, and wherein when the first solution and the second solution are mixed, a hydrogel is formed by electrostatic interaction.

More specifically, the injectable hydrogel composition consists of a first solution including anionic hyaluronic acid into which a vascular differentiation inducing factor is introduced; and a second solution including a cationic material,

In the present disclosure, the injectable hydrogel is formed by the electrostatic interaction of the anionic hyaluronic acid into which a vascular differentiation inducing factor is introduced and the cationic material. Preferably, when the first solution and the second solution are injected into the target site, they are mixed to form a hydrogel, but depending on the purpose, the first solution and the second solution are mixed immediately before injection to form a hydrogel and then injected.

The target site is a place requiring tissue regeneration, such as a damaged organ, a depressed tissue or a wound site, and the injectable hydrogel composition having the recruitment of endogenous progenitor cells or stem cells and the induction of vascular differentiation of the recruited cells according to the present disclosure has an advantage in that tissue can be regenerated more efficiently, because it recruits endogenous progenitor cells/stem cells with a hydrogel and induces the recruited progenitor cells/stem cells to differentiate into blood vessels.

In the present disclosure, the stem cells refer to endogenous progenitor cells or endogenous stem cells, and the endogenous progenitor cells or endogenous stem cells refer to cells with self-renewal and pluripotency that are originally present in a specific organ and/or tissue and contribute to the regeneration of the tissue and/or organ, when the corresponding organ and/or tissue is damaged. Specific examples include mesenchymal stem cells, corneal stem cells, myocardial stem cells, neural stem cells and vascular endothelial progenitor cells.

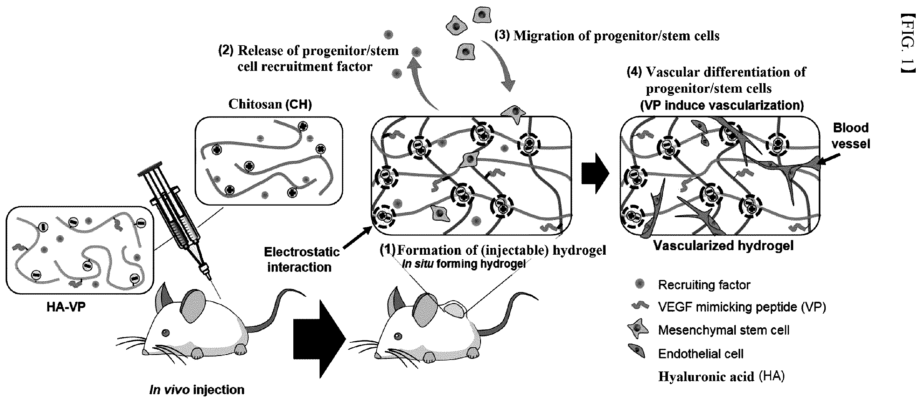

FIG. 1 is a mimetic diagram of the injectable hydrogel composition having the recruitment of endogenous progenitor cells or stem cells and the induction of vascular differentiation of the recruited cells according to the present disclosure, and in the present disclosure, the vascular endothelial growth factor mimic peptide (VP), which is capable of differentiating progenitor/stem cells into vascular cells, was chemically introduced into hyaluronic acid to prepare HA-VP in which VP was chemically introduced, and anionic HA-VP (first solution) and cationic chitosan (CH, second solution) were mixed to form a hydrogel through electrostatic interaction.

In order to physically support the progenitor cell/stem cell recruitment factor, substance P on the hydrogel, the progenitor cell/stem cell recruitment factor was mixed in each of the HA-VP solution and the chitosan solution, and the HA-VP solution and the chitosan solution including the progenitor/stem cell recruitment formed a hydrogel through electrostatic interaction when injected *in vivo.* The stem cell recruitment factor was released from the formed hydrogel, and endogenous progenitor cells/stem cells were recruited in the hydrogel, and the endogenous progenitor cells/stem cells recruited in the hydrogel differentiated into vascular cells by chemically introduced VP to promote the induction of angiogenesis.

In the present disclosure, the vascular differentiation inducing factor is a vascular endothelial growth factor mimic peptide includes the amino acid sequence of SEQ ID NO: 1 (KLTWQELYQLKYKGI), but is not limited thereto, and depending on the purpose, angiogenesis promoting factors such as vascular endothelial growth factor (VEGF), basic fibroblast growth factor (bFGF) and the like, or thrombin may be applied.

In the present disclosure, the anionic hyaluronic acid into which the vascular differentiation inducing factor is introduced is prepared by reacting the vascular differentiation inducing factor with anionic hyaluronic acid in which a carboxylic acid functional group is activated.

The molecular weight of the hyaluronic acid is preferably in the range of 500,000 Da to 6,000,000 Da. In this case, if the molecular weight of hyaluronic acid is less than 500,000 Da, the physical properties are too low to form a significant hydrogel, and if it is more than 6,000,000 Da, the viscosity may increase to bring a limitation to application as an injection, and thus, it is not preferable.

In the present disclosure, the cationic material is at least one selected from the group consisting of chitosan, cationic dextran, polyethyleneimine, polylysine and polyhistidine.

In the present disclosure, the stem cell recruitment is susbstance

In the present disclosure, the injectable hydrogel is formed through electrostatic interaction between anionic hyaluronic acid, into which the vascular differentiation inducing factor is introduced, and a cationic material.

In the present disclosure, the ratio of the first solution comprising anionic hyaluronic acid, into which the vascular differentiation inducing factor is introduced, and the second solution comprising a cationic material (chitosan) in the first solution is 2:1 to 1:2.

In the present disclosure, the storage modulus of the hydrogel formed by mixing the first solution and the second solution may be 10 to 100 Pa.

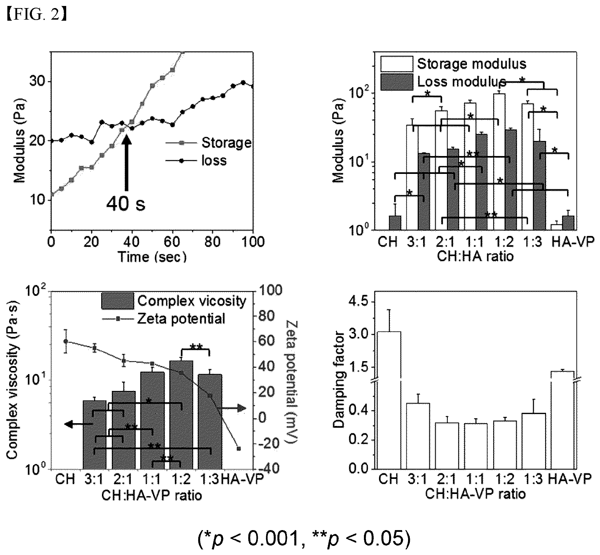

In a specific exemplary embodiment of the present disclosure, hyaluronic acid (HA-VP) into which the vascular endothelial growth factor mimic peptide was introduced was prepared by reacting hyaluronic acid in which a carboxylic acid functional group was activated and the vascular endothelial forming factor mimic peptide (Table 1 and Table 2), and when the chitosan (CH) solution and the HA-VP solution were mixed, it was confirmed that the chitosan and HA-VP formed a hydrogel through electrostatic interaction (FIG. 2).

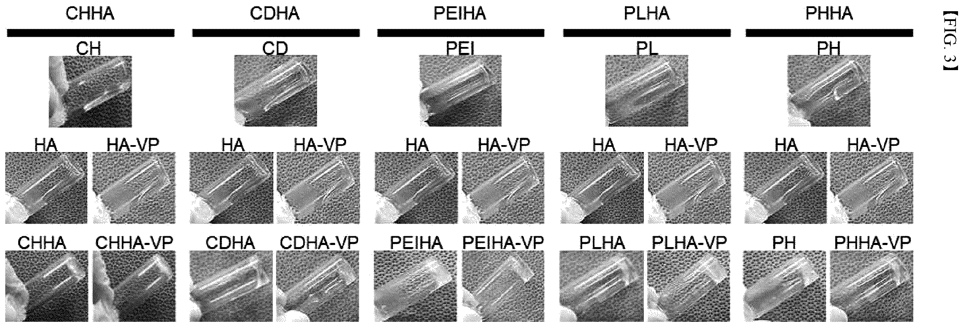

It was confirmed that the hydrogel was prepared through the electrostatic interaction of the cationic polymer such as chitosan (CH), cationic dextran (CD), polyethyleneimine (PEI), polylysine (PL) or poly histidine (PH) and the anionic HA or hyaluronic acid (HA-VP) into which the endothelial growth factor mimic peptide was introduced (FIG. 3).

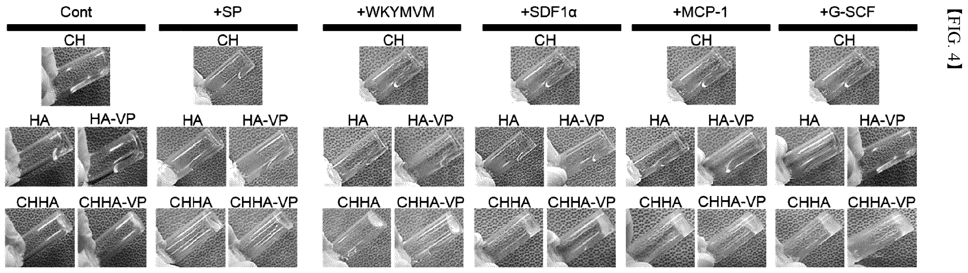

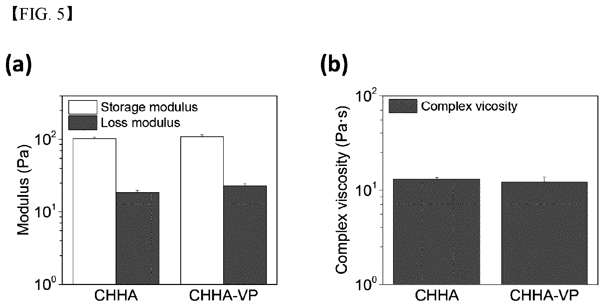

In addition, as a result of confirming whether the hydrogel formation is affected by the chemical introduction of VP and the presence or absence of a stem cell recruitment factor, it was confirmed that the hydrogel was formed through electrostatic attractive regardless of before and after the chemical introduction of VP and whether the progenitor cell/stem cell recruitment factor was mixed (FIG. 4), and it was confirmed that the introduction of VP did not significantly affect the rheological properties of the hydrogel (FIG. 5).

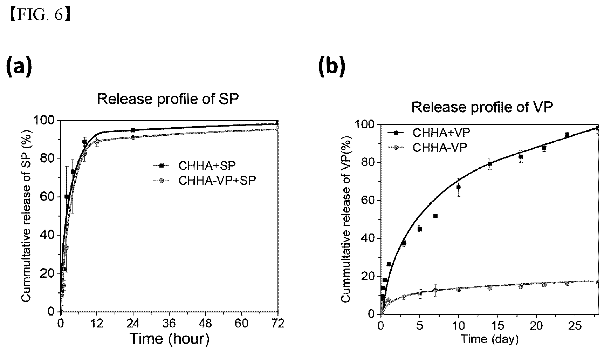

In another specific exemplary embodiment of the present disclosure, as a result of confirming the degree of the release of the stem cell recruitment factor and VP in the hydrogel prepared by electrostatic interaction, it was confirmed that the introduction of VP did not affect the characteristics of the hydrogel as a carrier, and when VP was chemically introduced, it had high sustained-release properties compared to the case where VP was simply mixed (FIG. 6).

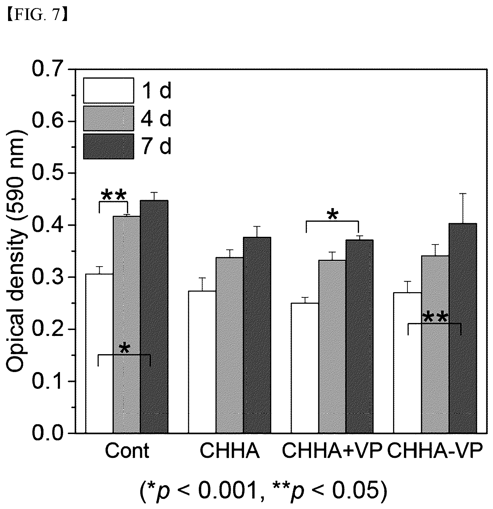

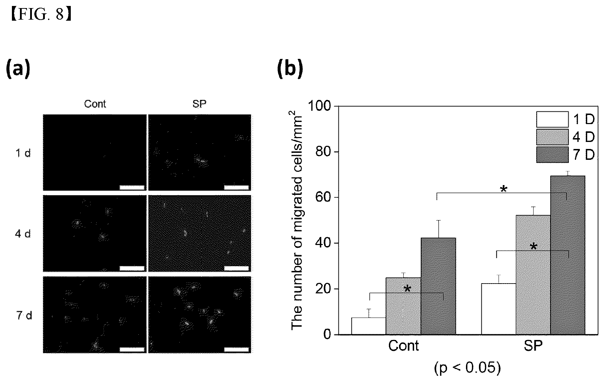

In addition, it was confirmed that the hydrogel of the present disclosure is nontoxic (FIG. 7), and it was confirmed that substance P, which is the stem cell recruitment factor, effectively recruits stem cells (FIG. 8).

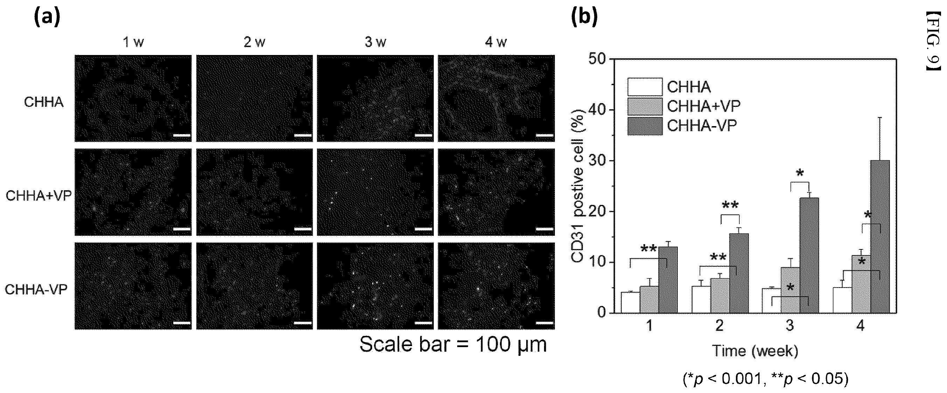

In still another specific exemplary embodiment of the present disclosure, as a result of confirming the angiogenesis effect by the hydrogel of the present disclosure *in vitro,* compared to the hydrogel (CHHA + VP) prepared by simply mixing the vascular endothelial growth factor mimic peptide with hyaluronic acid, the number of CD31-expressing stem cells was significantly increased in the hydrogel (CHHA-VP) prepared by chemically introducing the vascular endothelial growth factor mimic peptide into hyaluronic acid (FIG. 8), and it was confirmed that the expressions of the von Willebrand factor (vWF) gene and the CD31 gene expressed in vascular cells also significantly increased (FIG. 9).

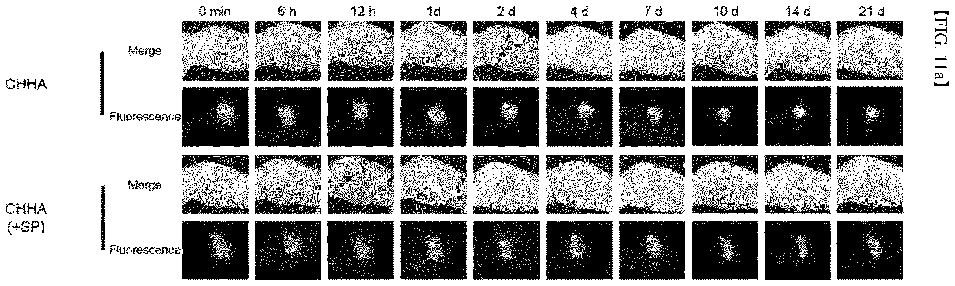

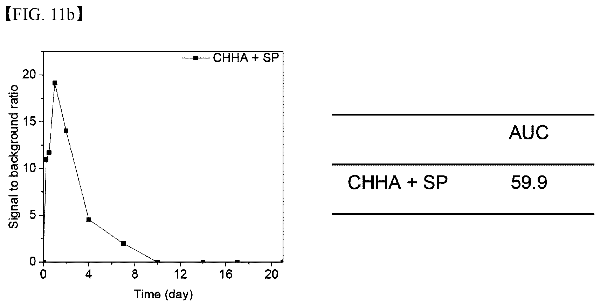

In still another specific exemplary embodiment of the present disclosure, as a result of confirming the ability to recruit stem cells by the hydrogel of the present disclosure *in vivo,* it was confirmed that the mesenchymal stem cells injected into the tail of mice were recruited toward the hydrogel by the stem cell recruitment factor included in the hydrogel (FIG. 11a and FIG. 11b).

As a result of confirming whether actual angiogenesis is induced *in vivo* by the hydrogel of the present disclosure, it was confirmed that the hydrogels maintained their shape well for 4 weeks, and more blood vessels were observed on the surface of the hydrogel when VP was chemically introduced (FIG. 12). In addition, compared to the hydrogel (CHHA+VP (+SP)) in which VP was simply mixed, it was confirmed that the recruited stem cells were observed in the hydrogel including the stem cell recruitment factor and chemically introduced with VP (CHHA-VP (+SP)), and the number of stem cells expressing CD31 increased (FIG. 13a and FIG. 13b), and it was also confirmed that the expression levels of the vWF gene and the CD31 gene were significantly increased (FIG. 14).

That is, it was confirmed that the injectable hydrogel composition having the recruitment of endogenous progenitor cells or stem cells and the induction of vascular differentiation of the recruited cells according to the present disclosure releases the stem cell recruitment factor from the injected hydrogel to thereby recruit endogenous progenitor cells/stem cells into the hydrogel, and the induction of angiogenesis was promoted by differentiating into vascular cells by the vascular differentiation inducing factor which was chemically introduced into the hyaluronic acid, and in particular, when the blood vessel differentiation inducing factor was chemically introduced into hyaluronic acid, it was confirmed that it exhibited a high angiogenesis-inducing effect.

Further, in the present disclosure, since endogenous progenitor cells/stem cells are recruited without injecting progenitor cells/stem cells from the outside, various side effects caused by injection into external cells may be solved. Therefore, since the hydrogel composition of the present disclosure can be used for tissue regeneration by utilizing endogenous progenitor cells/stem cells in the field of regenerative medicine, it can be applied to tissue improvement, wound treatment, scar improvement, skin tissue improvement, soft tissue connective correction, wrinkle removal or improvement, contour correction, tissue enlargement, breast enlargement or the like.

In another aspect, the present disclosure relates to an injection for tissue regeneration including the injectable hydrogel composition.

### [Advantageous Effects]

In the hydrogel composition of the present disclosure, it was confirmed that the stem cell recruitment factor was released from the injected hydrogel, and endogenous progenitor cells/stem cells were recruited in the hydrogel, and the induction of angiogenesis was promoted by differentiating into vascular cells by the vascular differentiation inducing factor chemically introduced into hyaluronic acid. In particular, it was confirmed that when the vascular differentiation inducing factor was chemically introduced into hyaluronic acid, a high angiogenesis-inducing effect was observed. Therefore, the hydrogel composition of the present disclosure has excellent recruitment of endogenous progenitor cells/stem cells and induction of vascular differentiation, and thus, it can be effectively applied to various tissue regenerations and wound treatments in addition to the formation of blood vessels.

### [Description of Drawings]

FIG. 1 is a mimetic diagram of the injectable hydrogel composition having the recruitment of endogenous progenitor cells or stem cells and the induction of vascular differentiation of the recruited cells according to the present disclosure.
FIG. 2 is data obtained by measuring the zeta potential and rheological properties according to the ratio of a cationic material (chitosan) and anionic hyaluronic acid (HA-VP) introduced with a vascular endothelial growth factor mimic peptide, and it shows (a) the modulus according to reaction time, (b) the modulus according to the ratio of chitosan (CH) and hyaluronic acid (HA), (c) the viscosity and zeta potential according to the ratio of chitosan and HA-VP, and (d) the damping factor according to the ratio of chitosan and HA-VP.
FIG. 3 is a set of images of hydrogels formed by electrostatic interaction, when cationic chitosan (CH), cationic dextran (CD), polyethyleneimine (PEI), polylysine (PL) or polyhistidine (PH) was mixed with an anionic hyaluronic acid (HA) solution or an HA-VP solution.
FIG. 4 is a set of images of hydrogels prepared through electrostatic interaction, and it is a set of images of hydrogels formed by electrostatic interaction, when a cationic chitosan (CH) solution with or without a progenitor/stem cell recruitment factor and an anionic hyaluronic acid (HA) solution or an HA-VP solution were mixed.
FIG. 5 is data obtained by measuring (a) the storage modulus/loss modulus and (b) the viscosity of a hydrogel (CHHA) prepared by reacting chitosan and hyaluronic acid and a hydrogel (CHHA-VP) prepared by reacting chitosan and HA-VP.
FIG. 6 is data obtained by measuring the release behaviors of (a) substance P and (b) VP in hydrogels formed by electrostatic interaction, when VP was simply mixed (CHHA + VP) or VP was chemically introduced (CHHA-VP) in hyaluronic acid.
FIG. 7 is data for evaluating the toxicity of the hydrogel.
FIG. 8 is data confirming the ability of substance P to recruit stem cells, showing (a) fluorescence microscopy observation images (red: stem cells) and (b) the number of migrated cells.
FIG. 9 is a set of images obtained by observing cells through immunostaining for CD31, in order to confirm the degree of induction of vascular differentiation by the hydrogel of the present disclosure *in vitro,* showing (a) fluorescence microscopy observation images and (b) the results of measuring the number of CD31-expressing cells.

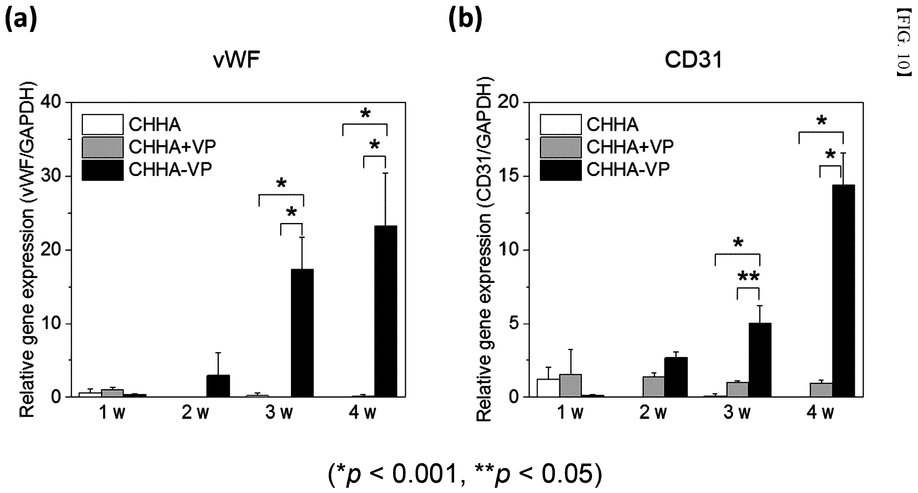

FIG. 10 is data confirming the changes in the gene expressions of (a) vWF and (b) CD31 *in vitro,* in order to confirm the degree of induction of vascular differentiation by the hydrogel of the present disclosure.
FIG. 11a is data confirming the stem cell recruitment of the hydrogel according to the presence or absence of a stem cell recruitment factor *in vivo,* and shows the results of observing the migration of human-derived mesenchymal stem cells (hMSC) through a fluorescence measuring device. Green indicates hydrogels and red indicates cells.
FIG. 11b is data confirming the stem cell recruitment of the hydrogel according to the presence or absence of a stem cell recruitment factor *in vivo,* and quantitatively shows the results of FIG. 11a.

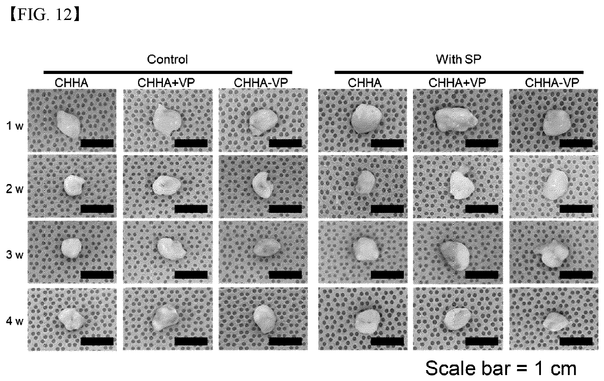

FIG. 12 is a set of images showing the appearance when the hydrogel injected *in vivo* was extracted in order to confirm the induction of angiogenesis by the hydrogel.

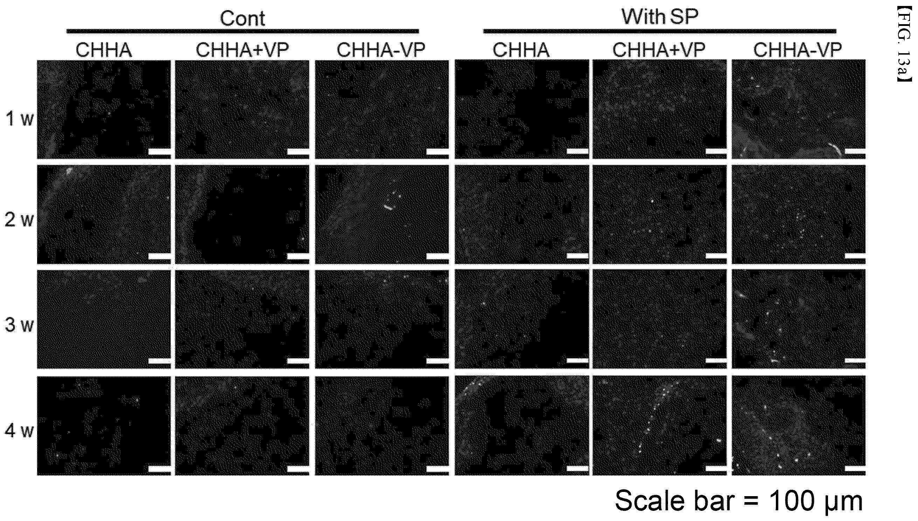

FIG. 13a shows images of immunostaining for BrdU and CD31 in the extracted hydrogel.

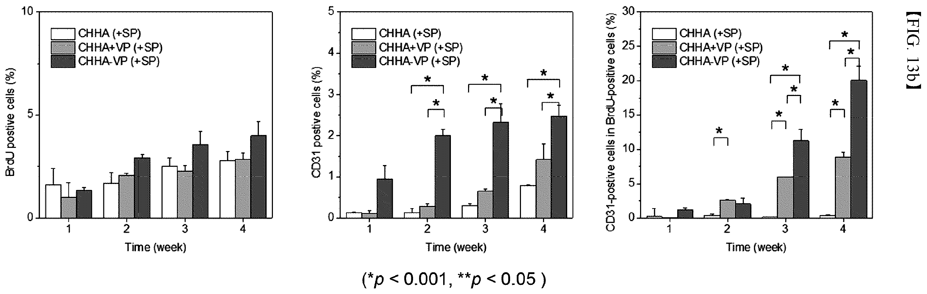

FIG. 13b shows data obtained by quantifying BrdU and CD31-positive cells in the extracted hydrogel.

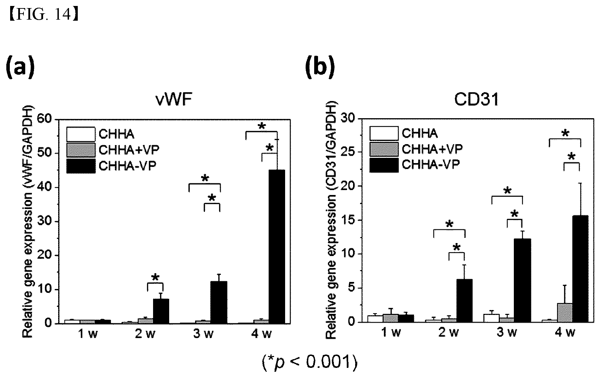

FIG. 14 is data confirming the angiogenesis effect of the extracted hydrogel through the changes in the gene expressions of (a) vWF and (b) CD31.

### [Modes of the Disclosure]

### [Example 1]

### Preparation of hyaluronic acid in which vascular endothelial growth factor mimic peptide is introduced

In the present disclosure, in order to prepare an injectable hydrogel composition having the recruitment of endogenous progenitor cells or stem cells and the induction of vascular differentiation of the recruited cells, first, hyaluronic acid into which a vascular endothelial growth factor mimic peptide was introduced was prepared.

Specifically, an HA solution was prepared by dissolving hyaluronic acid (HA) in distilled water at 10 mg/mL, and then 0.3 mg of 4-(4,6-dimethoxy-1,3,5-triazin-2-yl)-4-methylmorpholinium chloride (DMTMM, Sigma, USA) was added to 10 mL of the HA solution and stirred for 1 hour to activate the carboxylic acid functional group of hyaluronic acid.

10 mL of the HA solution in which the carboxylic acid functional group was activated was added dropwise to a VP solution prepared by dissolving 1.9 mg of the vascular endothelial growth factor mimic peptide (VP, SEQ ID NO: 1: KLTWQELYQLKYKGI) in 1 mL of distilled water, and it was reacted by stirring for 24 hours. The reaction solution was dialyzed for 72 hours and freeze-dried at -80°C to prepare HA-VP into which VP was introduced.

As shown in Tables 1 and 2 below, the VP introduced into the HA was confirmed through TNBSA analysis (Table 1) and elemental analysis (Table 2).

**[Table 1]**

| Sample | VP (µg/mg)_{cald} | VP (µg/mg)ₘₑₐₛ | Introduction ratio (%) |
|---|---|---|---|
| HA-VP | 19.1 | 16.0 | 84 |

**[Table 2]**

| Sample | C (%) | H (%) | N (%) | C/N mole ratio | DS (%), _{cald} | DS (%), _{EA} | Introductio n ratio (%) |
|---|---|---|---|---|---|---|---|
| HA | 34.5 | 5.7 | 2.8 | 14.45 | - | - | - |
| HA-VP | 38.3 | 5.7 | 3.2 | 13.86 | 0.40 | 0.34 | 85 |

### [Example 2]

### Measurement of zeta potential and rheological properties according to the ratio of chitosan and HA-VP

In the present disclosure, in order to confirm whether the HA-VP and chitosan prepared in Example 1 above formed a hydrogel through electrostatic interaction, the Zeta potential and rheological properties according to the ratio of chitosan and HA-VP were measured.

First, a CH solution was prepared by dissolving chitosan (CH, sigma, USA) in a 0.1 M aqueous acetic acid solution at a concentration of 20 mg/mL, and HA-VP was dissolved in distilled water at 20 mg/mL to prepare an HA-VP solution.

The CH solution and the HA-VP solution were mixed in proportions and the zeta potential of the hydrogel formed through electrostatic bonding was measured with ELS-Z (Otsuka Electronics, Japan), and the rheological properties were measured with a modular compact rheometer (MCR 102, Anton Paar, Austria). The measurement conditions for rheological properties were that the parallel plate had a diameter of 25 mm, the distance from the bottom surface was 0.3 mm, and the strain was 2% at 25°C and 1 Hz.

As a result, as shown in FIG. 2, when the CH solution and the HA-VP solution were mixed, a gelation point was observed in about 40 seconds, and it was confirmed that the zeta potential and the modulus changed according to the ratio. Through this, it was confirmed that chitosan and HA-VP formed an electrostatic bond. In addition, it was confirmed that the damping factor was less than 1 when both solutions were mixed, and through this, it was confirmed that chitosan and HA-VP formed a hydrogel through electrostatic interaction.

### [Example 3]

### Preparation of hydrogel through electrostatic interaction

### 3-1: Preparation of hydrogel according to the type of cationic material and anionic material

In the present disclosure, hydrogels were prepared by electrostatic interaction by respectively mixing the cationic polymer chitosan (CH), cationic dextran (CD), polyethyleneimine (PEI), polylysine (PL) or polyhistidine (PH) and anionic HA or hyaluronic acid (HA-VP) introduced with a vascular endothelial growth factor mimic peptide.

First, a CH solution was prepared by dissolving chitosan (CH, sigma, USA) in a 0.1 M aqueous acetic acid solution at a concentration of 20 mg/mL, and CD, PEI, PL, PH, HA and HA-VP were each dissolved in distilled water at 20 mg/mL to prepare a CD solution, a PEI solution, a PL solution, a PH solution, an HA solution and an HA-VP solution.

Then, hydrogels were prepared by mixing the CH solution, CD solution, PEI solution, PL solution or PH solution and HA solution, or the CH solution, CD solution, PEI solution, PL solution or PH solution and HA-VP solution in the same volume.

As a result, as shown in FIG. 3, it was confirmed that hydrogels were formed according to the electrostatic interaction between the cationic material and the anionic hyaluronic acid.

### 3-2: Preparation of hydrogel according to stem cell recruitment factor

A CHHA hydrogel or CHHA-VP hydrogel was prepared by mixing the CH solution and the HA solution prepared in Example 3-1 or the CH solution and the HA-VP solution in the same volume. In addition, one of SP, WKYMVM, SDF1α, G-SCF and MCP-1 (Genscript, USA) was dissolved in each of the CH, HA and HA-VP solutions having the same concentration at a concentration of 1 µg/mL, respectively, to prepare solutions, and it was carried out in the same way as forming the hydrogels previously.

**[Table 3]**

| Stem cell recruitment factor | | | |
|---|---|---|---|
| Classification | Protein | Peptide | Peptide sequence |
| Stem cell recruitment factor | | Substance P (SP) | RPKPQQFFGLM(SEQ ID NO: 2) |
| | | WKYMVM | WKYMVM(SEQ ID NO: 3) |
| | SDF1 | | |
| | MCP-1 | | |
| | G-SCF | | |

Examples relating to WKYMV, SDF1, MCP-1 and G-SCF are not according to the claimed invention.

As shown in FIG. 4, it was confirmed that the hydrogels were formed through electrostatic interaction regardless of before and after the chemical introduction of VP and the presence or absence of mixed progenitor cells/stem cell recruitment factors.

### [Example 4]

### Evaluation of rheological properties of hydrogel

In order to evaluate the rheological properties of the CHHA and CHHA-VP hydrogels prepared in Example 3 above, the modulus and viscosity were measured using a modular compact rheometer (MCR 102, Anton Paar, Austria). In this case, the used parallel plate had a diameter of 25 mm, an interval from the bottom surface of 0.3 mm, and a strain of 2% at 25°C and 1 Hz.

As a result, as shown in FIG. 5, it was confirmed that CHHA and CHHA-VP had equal levels of storage modulus and loss modulus, and also exhibited equal levels of viscosity. Therefore, it can be seen that the introduction of VP did not significantly affect the rheological properties of the hydrogels.

### [Example 5]

### Confirmation of release behavior of stem cell recruitment factor and VP in vitro

In the present disclosure, it was attempted to confirm the release degree of the stem cell recruitment factor and VP in the hydrogels prepared by electrostatic interaction.

First, an HA+VP solution was prepared by simply mixing VP with the HA solution prepared in Example 3 at a concentration of 320 µg/mL, and SP, which is a stem cell recruitment factor, was mixed with each of the HA solution and HA-VP solution at a concentration of 2 µg/mL to prepare an HA+SP solution and HA-VP+SP solution, respectively.

Next, 100 µL of the CH solution and 100 µL of the HA+VP solution were mixed in a vial to prepare 200 µL of a CHHA+VP hydrogel, and 100 µL of the CH solution and 100 µL of the HA-VP solution were mixed in a vial to prepare 200 µL of a CHHA-VP hydrogel.

In addition, 100 µL of the CH solution and 100 µL of HA+SP solution were mixed in a vial to prepare 200 µL of a CHHA+SP hydrogel, and 100 µL of the CH solution and 100 µL of the HA-VP+SP solution were mixed in a vial to prepare 200 µL of a CHHA-VP + SP hydrogel.

3 mL of a physiological saline was placed in each vial and stored in an incubator at 100 rpm and 37°C, and after taking 1 mL of the physiological saline from the vial at a predetermined time, 1 mL of a new physiological saline was added to the vial, and it was carried it out for 28 days to conduct a release experiment.

As a result, as shown in FIG. 6(a), it was confirmed that the release of SP occurred regardless of the chemical introduction of VP, and in other words, it was confirmed that the introduction of VP did not affect the properties of the hydrogel as a carri er.

In addition, as shown in FIG. 6(b), almost all VP was released at the beginning of the release experiment in the release result of VP when VP was simply mixed, whereas when VP was chemically introduced like CHHA-VP, it was confirmed that it was hardly released inside the hydrogel. That is, it was confirmed that the chemically introduced VP had higher sustained-release properties compared to the simple mixing of VP.

### [Example 6]

### Toxicity evaluation of hydrogel

The presence or absence of toxicity of the hydrogels prepared in the present disclosure was evaluated.

First, human mesenchymal stem cells (hMSCs) were mixed at a concentration of 1 × 10⁶ cells/mL in each of the CH, HA, HA-VP solutions prepared in Example 3 above and the HA+VP solution prepared in Example 5 above.

Cytotoxicity evaluation test was performed for the prepared solutions, and in order to form hydrogels, (1) 200 µL of the CH solution and 200 µL of the HA solution were mixed to form 400 µL of a CHHA hydrogel, (2) 200 µL of the CH solution and 200 µL of the HA+VP solution were mixed to form 400 µL of a CHHA+VP hydrogel, and (3) 200 µL of the CH solution and 200 µL of the HA-VP solution were mixed to form 400 µL of a CHHA-VP hydrogel in 24-well plates. As a control group for comparison, 4×10⁵ hMSC cells were added to a 24-well plate. The medium was added by 1 mL and was replaced every 3 days, and cytotoxicity was measured by MTT analysis on days 1, 4 and 7.

As a result, as shown in FIG. 7, it was confirmed that no significant toxicity was observed in all hydrogel groups compared to the control group.

### [Example 7]

### Confirmation of SP's ability to recruit stem cells

In the present disclosure, the ability of the SP to recruit stem cells was confirmed.

First, human-derived mesenchymal stem cells (hMSCs) were labeled with PKH 26 dye (Sigma, USA) and then dispensed in the upper chamber (8.0 µm pore size) of a 24-well trans well plate (SPL, Korea) to be 5 × 10⁴ cells. After culturing in the upper chamber for 48 hours using serum-free DMEM (Dubelco's modified eagle medium, Gibco, USA) medium, DMEM containing 1 µg/mL SP and 1% FBS (fetal bovine serum, Gibco, USA) was added to the bottom well, and the medium was replaced every 3 days, and the hMSCs that migrated to the bottom well were observed using a fluorescence microscope (Olympus, Japan). As a control group, DMEM (including 1% FBS) without SP was added to the bottom well and observed.

As a result, as shown in FIG. 8, it was confirmed that more cells migrated to the lower side when SP was present than in the case when SP was absent, and through this, it was confirmed that the SP recruited stem cells. [Table 4] below is the results of quantitative analysis of the data confirming the stem cell recruitment of substance P.

**[Table 4]**

| | Migration rate of hMSC (cells/day) | Rate constant (1/day) | Relative migration rate |
|---|---|---|---|
| Cont | 1.1×10³ | -0.47 | - |
| SP | 1.9×10³ | -0.49 | 1.7 |

### [Example 8]

### Confirmation of angiogenesis effect by the hydrogel of the present disclosure in vitro

### 8-1: Confirmation of angiogenesis through immunofluorescence analysis

In order to confirm the angiogenesis-inducing effect of the injectable hydrogel of the present disclosure, CHHA, CHHA+VP and CHHA-VP hydrogels including human-derived mesenchymal stem cells were prepared in the same manner as in Example 6, respectively, and the medium was exchanged every 3 days and cultured for 4 weeks.

At the 1^{st}, 2^{nd}, 3^{rd} and 4^{th} weeks of culture, the hydrogel was fixed with formalin, and immunofluorescence analysis was performed to observe the expression of CD31, which is known to be expressed in vascular cells.

As a result, as shown in FIG. 9, compared to the hydrogel (CHHA+VP) prepared by simply mixing the vascular endothelial growth factor mimic peptide with hyaluronic acid, it was confirmed that the number of stem cells expressing CD31 was significantly increased in the hydrogel (CHHA-VP) prepared by chemically introducing the vascular endothelial growth factor mimic peptide into hyaluronic acid.

### 8-2: Confirmation of angiogenesis through gene expression change

After extracting mRNA from the hydrogels of Example 8-1, cDNA was synthesized using the extracted mRNA, and changes in the expressions of the von Willebrand factor (vWF) gene and the CD31 gene expressed in vascular cells were confirmed through qRT-PCR (quantitative real-time polymerase chain reaction).

**[Table 5]**

| Primer sequence | | | |
|---|---|---|---|
| Gene | Base sequence (5'->3') | | SEQ ID NO |
| vWF | Forward | CGG CTT GCA CCA TTC AGC TA | SEQ ID NO: 4 |
| | Reverse | TGC AGAAGT GAG TAT CAC AGC CAT C | SEQ ID NO: 5 |
| CD31 | Forward | ATT GCA GTG GTT ATC ATC GGA GTG | SEQ ID NO: 6 |
| | Reverse | CTC GTT GTT GGA GTT CAG AAG TGG | SEQ ID NO: 7 |
| GAPDH | Forward | GAA GGT GAA GGT CGG AGT C | SEQ ID NO: 8 |
| | Reverse | GAA GAT GGT GAT GGG ATT TC | SEQ ID NO: 9 |

As a result, as shown in FIG. 10, when VP was chemically introduced, it was confirmed that the expressions of the vWF gene and the CD31 gene were higher than when there was no VP and when VP was simply mixed. That is, it was confirmed that the hydrogel of the present disclosure effectively induces differentiation into vascular cells.

### [Example 9]

### Confirmation of recruitment of stem cells by hydrogel in vivo

It was confirmed whether the actual stem cells were recruited *in vivo* by the hydrogel of the present disclosure.

First, for fluorescence imaging, 1 µg/mL of SP, which is a stem cell recruitment factor, was added to each of an FITC-labeled chitosan (CH) solution and a hyaluronic acid (HA) solution, and a total of 200 µL of the hydrogel by 100 µL each was injected subcutaneously in nude mice through a dual syringe.

Afterwards, 1 × 10⁶ hMSCs labeled with IR-783 were injected into the tail vein of nude mice, and cell migration was observed through fluorescence imaging. The results are shown in FIG. 10, and the hydrogel is shown in green color and the cell is shown in red color.

As a result, as shown in FIG. 11a and FIG. 11b, red fluorescence by the cells was not observed in the absence of the stem cell recruitment factor, and red fluorescence by the cells was observed in the presence of the recruitment factor. That is, it was confirmed that the stem cells were recruited toward the hydrogel by the stem cell recruitment factor.

### [Example 10]

### Confirmation of induction of angiogenesis by hydrogel in vivo

### 10-1: Preparation and injection of hydrogel

It was confirmed whether actual angiogenesis was induced *in vivo* by the hydrogel of the present disclosure.

First, 1 µg/mL of SP, which is a stem cell recruitment factor, was added to the CH, HA, HA+VP, and HA-VP solutions, respectively, and then, a total of 200 µL of the hydrogel by 100 µL each was injected subcutaneously in nude mice through a dual syringe in the combination of CH and HA, CH and HA+VP, and CH and HA-VP. For comparison, the CH, HA, HA+VP and HA-VP solutions without the recruitment factor were injected in the same way. Afterwards, 1 × 10⁶ hMSCs labeled with BrdU were injected into the tail vein of nude mice. The hydrogel was extracted at the 1^{st}, 2^{nd}, 3^{rd} and 4^{th} weeks, and it was observed whether angiogenesis occurred.

As a result, as shown in FIG. 12, it was confirmed that the hydrogels maintained their shape well for 4 weeks, and more blood vessels were observed on the surface of the hydrogel when VP was chemically introduced.

### 10-2: Confirmation of angiogenesis through immunofluorescence analysis

Immunofluorescence analysis was performed on BrdU and CD31 to confirm the degrees of hMSCs recruited in the hydrogels extracted in Example 10-1 and angiogenesis. The observation results are shown in FIG. 13a and FIG. 13b, and BrdU was stained with red, and CD31 was stained with green.

As a result, as shown in FIG. 13a and FIG. 13b, no red color was observed in the absence of the recruitment factor, which means that hMSCs were not recruited. On the other hand, when the recruitment factor was included, red-stained cells were observed inside the hydrogel, and through this, it was confirmed that the hMSCs were recruited inside the hydrogel.

In the case of CD31 stained green, it was confirmed that the number of cells expressing CD31 was increased when VP was chemically introduced, compared to when VP was physically mixed.

Further, in the case of cells stained with BrdU and CD31 at the same time, it was confirmed that it was increased more in the hydrogel in which VP was chemically introduced, and through this, it was confirmed that the differentiation into vascular cells and the promotion of angiogenesis were more effectively induced in the presence of the recruitment factor by VP which was chemically introduced and recruited hMSCs inside the hydrogel than VP which was simply mixed.

### 10-3: Confirmation of angiogenesis through gene expression change

The mRNA was extracted from the hydrogels extracted in Example 10-1, and qRT-PCR was performed in the same manner as in Example 8-2.

As a result, as shown in FIG. 14, it was confirmed that the vWF gene and CD31 gene, which are expressed in human cells, were expressed in the hydrogels extracted from nude mice, and through this, it was confirmed that hMSC, which were injected into the tail veil, migrated by the recruitment factor and were recruited in the hydrogel. In addition, when VP was chemically introduced, it was confirmed that the expression levels of the vWF gene and the CD31 gene were significantly increased compared to the cases where there was no VP and VP was simply mixed, and through this, it was confirmed that angiogenesis was induced by the hydrogel of the present disclosure.

### [Industrial Applicability]

In the hydrogel composition having the recruitment of endogenous progenitors or stem cells and the induction of vascular differentiation of recruited cells according to the present disclosure, it was confirmed that the stem cell recruitment factor was released from the injected hydrogel, and endogenous progenitor cells/stem cells were recruited in the hydrogel, and the induction of angiogenesis was promoted by differentiating into vascular cells by the vascular differentiation inducing factor chemically introduced into hyaluronic acid. In particular, it was confirmed that when the vascular differentiation inducing factor was chemically introduced into hyaluronic acid, a high angiogenesis-inducing effect was observed. Therefore, the hydrogel composition of the present disclosure has excellent recruitment of endogenous progenitor cells/stem cells and induction of vascular differentiation, and thus, since it can be effectively applied to various tissue regenerations and wound treatments in addition to the formation of blood vessels, it has high industrial applicability.

### [Sequence List Free Text]

SEQ ID NO: 1 shows the amino acid sequence of the vascular endothelial growth factor mimic peptide, which is a vascular differentiation inducing factor.
SEQ ID NO: 2 shows the amino acid sequence of the substance P (SP) peptide, which is a stem cell recruitment factor.
SEQ ID NO: 3 shows the amino acid sequence of the WKYMVM peptide, which is a stem cell recruitment factor.
SEQ ID NO: 4 shows the nucleotide sequence of the forward primer of vWF.
SEQ ID NO: 5 shows the nucleotide sequence of the reverse primer of vWF.
SEQ ID NO: 6 shows the nucleotide sequence of the forward primer of CD31.
SEQ ID NO: 7 shows the nucleotide sequence of the reverse primer of CD31.
SEQ ID NO: 8 shows the nucleotide sequence of the forward primer of GAPDH.
SEQ ID NO: 9 shows the nucleotide sequence of the reverse primer of GAPDH.

## Claims

1. An injectable hydrogel composition having the recruitment of endogenous progenitors or stem cells and the induction of vascular differentiation of recruited cells, consisting of:
a first solution comprising anionic hyaluronic acid into which a vascular differentiation inducing factor is introduced, which is prepared by reacting the vascular differentiation inducing factor and anionic hyaluronic acid in which a carboxylic acid functional group is activated; and
a second solution comprising a cationic material, at least one selected from the group consisting of chitosan, cationic dextran, polyethyleneimine, polylysine and polyhistidine,
wherein a stem cell recruitment factor, substance P, is further comprised in any one or more of the first solution and the second solution,
wherein when the first solution and the second solution are mixed in ratio of 2:1 to 1:2, a hydrogel is formed by electrostatic interaction,
wherein the vascular differentiation inducing factor is a vascular endothelial cell growth factor mimic peptide, and
wherein the vascular endothelial cell growth factor mimic peptide comprises the amino acid sequence represented by SEQ ID NO: 1.

2. The injectable hydrogel composition of claim 1, wherein the storage modulus of the hydrogel formed by mixing the first solution and the second solution is 10 to 100 Pa.

3. The injectable hydrogel composition according to either claim 1 or claim 2, for use in tissue regeneration.

## Patentansprüche

1. Injizierbare Hydrogelzusammensetzung mit der Rekrutierung endogener Vorläufer oder Stammzellen und der Induktion von vaskulärer Differenzierung rekrutierter Zellen, bestehend aus:
einer ersten Lösung, die anionische Hyaluronsäure, in die ein vaskulärer Differenzierungsinduzierungsfaktor eingeführt wird, der durch Reagierenlassen des vaskulären Differenzierungsinduzierungsfaktors hergestellt wird, und anionische Hyaluronsäure umfasst, in der eine funktionelle Carbonsäuregruppe aktiviert ist; und
einer zweiten Lösung, die ein kationisches Material, mindestens eines ausgewählt aus der Gruppe bestehend aus Chitosan, kationischem Dextran, Polyethylenimin, Polylysin und Polyhistidin, umfasst,
wobei ein Stammzellrekrutierungsfaktor, Substanz P, ferner in einer oder mehreren beliebigen aus der ersten Lösung und der zweiten Lösung umfasst,
wobei, wenn die erste Lösung und die zweite Lösung im Verhältnis von 2:1 bis 1:2 gemischt werden, ein Hydrogel durch elektrostatische Interaktion gebildet wird,
wobei der vaskuläre Differenzierungsinduzierungsfaktor ein vaskuläres endotheliales Zellwachstumsfaktor-Mimikpeptid ist, und
wobei das vaskuläre endotheliale Zellwachstumsfaktor-Mimikpeptid die Aminosäuresequenz umfasst, die durch SEQ ID NO:1 dargestellt ist.

2. Injizierbare Hydrogelzusammensetzung nach Anspruch 1, wobei der Speichermodul des Hydrogels, das durch Mischen der ersten Lösung und der zweiten Lösung gebildet wird, 10 bis 100 Pa beträgt.

3. Injizierbare Hydrogelzusammensetzung nach Anspruch 1 oder Anspruch 2 zur Verwendung bei der Geweberegeneration.

## Revendications

1. Composition d'hydrogel injectable présentant le recrutement de cellules progénitrices ou de cellules souches endogènes et l'induction d'une différenciation vasculaire des cellules recrutées, comprenant :
une première solution comprenant de l'acide hyaluronique anionique où est introduit un facteur induisant une différenciation vasculaire, qui est préparée en faisant réagir le facteur induisant une différenciation vasculaire et de l'acide hyaluronique anionique où est activé un groupe fonctionnel acide carboxylique ; et
une seconde solution comprenant un matériau cationique, au moins un élément choisi dans le groupe comprenant le chitosane, le dextrane cationique, la polyéthylèneimine, la polylysine et la polyhistidine,
dans laquelle un facteur de recrutement de cellules souches, la substance P, est en outre compris dans une ou plusieurs quelconques solutions parmi la première solution et la seconde solution,
dans laquelle, lorsque la première solution et la seconde solution sont mélangées dans un rapport de 2:1 à 1:2, un hydrogel est formé par interaction électrostatique,
dans laquelle le facteur induisant une différenciation vasculaire est un peptide mimétique de facteur de croissance de cellules endothéliales vasculaires, et
dans laquelle le peptide mimétique de facteur de croissance de cellules endothéliales vasculaires comprend la séquence d'acides aminés représentée par SEQ ID N° : 1.

2. Composition d'hydrogel injectable de la revendication 1, dans laquelle le module de stockage de l'hydrogel formé en mélangeant la première solution et la seconde solution est de 10 à 100 Pa.

3. Composition d'hydrogel injectable selon soit la revendication 1 soit la revendication 2, destinée à être utilisée dans la régénération tissulaire.
